# EUROPÄISCHE PATENTANMELDUNG

(11) **EP 3 662 782 A1**
(43) Veröffentlichungstag der Anmeldung: **10.06.2020**
(21) Anmeldenummer: 19207453.2
(22) Anmeldetag: 06.11.2019
(51) Int. Cl.: A45D 34/04, A61M 5/30, A61N 5/06

(54) **ELEKTRISCHES HANDGERÄT ZUM EINBRINGEN VON KOSMETISCHEN PRÄPARATEN IN DIE HAUT**

(30) Priorität: 07.11.2018 DE 102018127795
(71) Anmelder: Bode, Jutta, 65239 Hohenstein (DE); Bode, Lothar, 65329 Hohenstein (DE)
(72) Erfinder: Bode, Gero, 65239 Hohenstein (DE)
(74) Vertreter: Blumbach · Zinngrebe Patentanwälte PartG mbB

(57) **Zusammenfassung**

Die Erfindung betrifft ein elektrisches Handgerät (10) zum Einbringen von kosmetischen Präparaten in die Haut, welches insbesondere für den Hausgebrauch bestimmt ist.

Das elektrische Handgerät (10) weist ein Gehäuse (11) und eine auf eine zu behandelnde Hautstelle aufsetzbare Düse (15) auf, wobei in dem Gehäuse (11)
eine elektrisch ansteuerbare Pumpe (50),
ein elektrisch ansteuerbares Ventil (40), welches über einen ersten Druckmittelkanal (31) mit der elektrischen Pumpe (50) verbunden ist,
mehrere im Gehäuse (11) angeordnete lichtemittierende Bauelemente (200), die zum Erzeugen von blauem Licht ausgebildet sind,
ein zweiter Druckmittelkanal (30), und
eine Steuereinheit (110) zum Ansteuern des Ventils (40) und der Pumpe (50) angeordnet sind, und wobei
die Düse (15) über den zweiten Druckmittelkanal (30) mit dem elektrisch ansteuerbaren Ventil (40) verbunden ist, wobei ein transluzentes Material (13) zur Farblichtbehandlung der zu behandelnden Haut in einer ringförmigen Aussparung des Gehäuses (11) angeordnet ist, und wobei die Steuereinheit (110) zum Ansteuern der lichtemittierenden Bauelemente (200) ausgebildet ist.

## Beschreibung

Die Erfindung betrifft ein elektrisches Handgerät zum Einbringen von kosmetischen Präparaten in die Haut, welches insbesondere für den Hausgebrauch bestimmt ist.

Es ist üblich, bei der Schönheitspflege vornehmlich der menschlichen Haut, eine Körperlotion oder die verschiedenartigsten Cremes auf die äußere Zellschicht der Haut, die sogenannte Epidermis, aufzutragen und je nach Anwendungsfall mehr oder weniger intensiv einzureiben. Die Wirkstoffe der aufgetragenen Lotion oder Creme werden teilweise direkt in die Zellschicht eingerieben oder diffundieren langsam dort hinein. Auf dem Gebiet der Kosmetik sind darüber hinaus Techniken im Einsatz, mit denen die Kosmetika auf die zu behandelnde Hautoberfläche aufgesprüht werden. Diesen Behandlungstechniken haftet der Nachteil an, dass die auf der Hautoberfläche aufgetragenen und eingeriebenen oder aufgesprühten Substanzen nur teilweise in die Epidermis eindiffundieren. Der Rest verflüchtigt sich in die Umgebung.

Aus der DE 102 52 917 A1 ist ein Handgerät zum Einbringen einer Substanz in eine Hautstelle bekannt, welches einen gehäuseförmigen Grundkörper aufweist, in dem ein Druckmittelbehälter zum Bereitstellen eines gasförmigen oder flüssigen Druckmittels, ein steuerbares Absperrventil und ein dem Druckmittelbehälter zugeordneter Druckminderer, der einen vorbestimmten, einstellbaren Druck an seinem Ausgang liefert, angeordnet sind. Ferner weist das Handgerät eine auf eine zu behandelnde Hautstelle aufsetzbare Düse auf, die über eine, vorzugsweise vollständig innerhalb des Grundkörpers verlaufende Druckmittelverbindung mit dem Druckmittelbehälter verbunden ist.

In der WO 2006/086742 A2 wird ein nadelloser Injektor beschrieben, mit welchem eine flüssige, einen biologisch aktiven Wirkstoff enthaltene Formulierung mittels eines flüssigen Mikrostroms unter hohem Druck in eine gewünschte Gewebetiefe unter der Haut eingebracht werden kann. Der erforderliche Druck wird mittels einer MEMS-Pumpe, die im Injektor angeordnet ist, erzeugt.

Der Wunsch nach glatter Haut ohne Nadelinjektionen, Schmerzen und Schwellungen ist die Basis für die Suche nach effektiven, sanften und gleichzeitig sicheren ästhetischen Behandlungsmethoden. Die Zellzwischenräume der Haut dienen als natürlich gegebene Kanäle, über die das Einschleusen kosmetischer Wirkstoffe tief in die Haut möglich ist. Der aus der WO 2006/086742 A2 bekannte Injektor arbeitet mit Druckintensitäten, die einen Schusskanal in der Haut verursachen, nicht schmerzfrei sind und die Haut verletzen.

Der Erfindung liegt daher die Aufgabe zugrunde, ein elektrisches Handgerät zum Einbringen von kosmetischen Präparaten in die Haut zu schaffen, welches eine effektive, sanfte und schmerzfreie Behandlung der Haut ermöglicht. Ein weiterer Aspekt der Erfindung kann darin gesehen werden, ein elektrisches Handgerät zu schaffen, welches sicher und zuverlässig in der Anwendung und kostengünstiger in der Wartung ist, sowie weniger Verschleißteile enthält, die entsorgt werden müssen.

Ein Kerngedanke der Erfindung kann darin gesehen werden, ein elektrisches Handgerät zum Einbringen von kosmetischen Präparaten in die Haut zu schaffen, welches eine multiple und flexible Hautbehandlung durch eine Kombination aus Druckinjektion und Farblichtbehandlung ermöglicht. Bei diesem Handgerät werden ferner keine Umwelt belastenden, durch den Hochdruck potentiell gefährlichen und regelmäßig auszutauschenden Druckmittelbehälter eingesetzt. Zudem ist eine sanfte, schmerzfreie, hautberuhigende und entzündungshemmende Hautbehandlung mit einem einzigen Handgerät ermöglicht. Ein derartiges elektrisches Handgerät ist kompakt, leicht handhabbar und kann auch sicher und zuverlässig von Privatpersonen zu Hause verwendet werden.

Das oben genannte technische Problem wird durch die Merkmale des Anspruchs 1 gelöst.

Demnach wird ein elektrisches Handgerät zum Einbringen von kosmetischen Präparaten in die Haut bereitgestellt, welches ein Gehäuse und eine auf eine zu behandelnde Hautstelle aufsetzbare Düse aufweist. In dem Gehäuse sind eine elektrisch ansteuerbare Pumpe, ein elektrisch ansteuerbares Ventil, welches über einen ersten Druckmittelkanal mit der elektrischen Pumpe verbunden ist, ein zweiter Druckmittelkanal und eine Steuereinheit zum Ansteuern des Ventils und der Pumpe angeordnet, wobei die Düse über den zweiten Druckmittelkanal mit dem elektrisch ansteuerbaren Ventil verbunden ist. Die Düse kann zum Beispiel an dem Gehäuse angeformt oder lösbar am Gehäuse befestigt sein. Zur zusätzlichen Farblichtbehandlung der zu behandelnden Haut, ist ein transluzentes Material, vorzugsweise in Ringform, vorgesehen, welches in einer entsprechenden Aussparung des Gehäuses angeordnet ist. Zur Beleuchtung des transluzenten Materials können mehrere lichtemittierende Bauelemente im Gehäuse angeordnet sein, wobei die Steuereinheit zum Ansteuern der lichtemittierenden Bauelemente ausgebildet ist. Bei den lichtemittierenden Bauelementen kann es sich zum Beispiel um LEDs handeln, die je nach Implementierung und Ansteuerung Licht in verschiedenen Wellenlängen aussenden können.

Vorteilhafterweise ist das transluzente Material derart geformt, dass blaues Licht, welches von den lichtemittierenden Bauelementen innerhalb des Gehäuses erzeugt wird, insbesondere nach vorne, d.h. in Richtung zur Düse auf die zu behandelnde Haut abgestrahlt wird.

Die lichtemittierenden Bauelemente erzeugen vorzugsweise ein blaues Farblicht mit einer Wellenlänge von beispielsweise 470nm, welches über das transluzente Material zur Hautberuhigung und zur Erhöhung der Behandlungswirkung u.a. in Richtung der zu behandelnden Haut ausgestrahlt wird. Blaues Farblicht wirkt hautberuhigend und entzündungshemmend. Auf diese Weise wird die Haut bei der Druckinjektion sofort beruhigt und kann sich somit schneller regenerieren.

Vorteilhafte Weiterbildungen sind Gegenstand der Unteransprüche.

Eine effiziente Farblichtbehandlung kann dadurch erzielt werden, dass der zweite Druckmittelkanal aus einem transluzenten Material hergestellt ist, so dass das von den lichtemittierenden Bauelementen ausgesendete blaue Licht durch die Düse direkt auf die zu behandelnde Haut geleitet wird.

Der erste und zweite Druckmittelkanal können zum Beispiel jeweils als Schlauch ausgebildet sein, wobei vorzugsweise zumindest der zweite Druckmittelkanal als transluzenter Schlauch ausgebildet ist.

Um ein sicheres und zuverlässiges Betreiben des elektrischen Handgeräts sicherzustellen, kann eine Temperaturüberwachungseinrichtung, die elektrisch mit der Steuereinheit verbunden ist, im Gehäuse implementiert sein. Sie dient insbesondere dazu, die Temperatur der elektrischen Pumpe zu überwachen, um ein Überhitzen der Pumpe verhindern zu können.

Die Temperarturüberwachungseinrichtung kann ein Temperatursensor, der die gemessene Temperatur regelmäßig der Steuereinheit zur weiteren Veranlassung überträgt, oder ein selbsttätig rückstellender Temperaturschalter sein, der bei Überschreiten einer Temperaturschwelle anspricht. Eine Zustandsänderung des Temperaturschalters wird von der Steuereinheit erfasst.

Zur Eingabe von Steuerbefehlen kann ein Bedienfeld vorgesehen sein, welches elektrisch mit der Steuereinheit verbunden ist und in dem zusätzlich zur optischen Unterstützung des Anwenders lichtemittierende Bauelemente, beispielsweise LEDs, in den Bedienfeld-Tastern angeordnet sein können.

Zur akustischen Unterstützung eines Anwenders kann ein akustischer Signalgeber vorgesehen sein, der elektrisch mit der Steuereinheit verbunden ist. Der akustische Signalgeber kann als Piezo-Signalgeber ausgebildet sein.

Um zum Beispiel einer Überhitzung des Handgerätes durch eine zu lange, nicht bestimmungsgemäße Anwendungsdauer durch den Anwender entgegenzuwirken, kann beispielsweise die Steuereinheit die Funktion eines Zeitgebers ausführen, die ermöglicht, dass das Handgerät, insbesondere die elektrisch ansteuerbare Pumpe, nach einer bestimmten Anwendungsdauer automatisch abgeschaltet werden kann.

Um den Zellverbund in der zu behandelnden Haut zu lockern, kann im Gehäuse ein Vibrationsmotor angeordnet sein, der dazu ausgebildet ist, die Düse unmittelbar oder mittelbar in Schwingung zu versetzen. Auf diese Weise können Präparate noch besser in die Haut eindiffundieren. Der Vibrationsmotor kann hierzu mit der Düse, dem zweiten Druckmittelkanal oder dem transluzenten Material in körperlichem Kontakt stehen.

Um zum Beispiel ein Überhitzen des elektrisch ansteuerbaren Ventils verhindern zu können, kann die Steuereinheit dazu ausgebildet sein, das elektrisch ansteuerbare Ventil mittels Pulsweitenmodulation anzusteuern.

Um zum Beispiel einen möglichen Versuch des Anwenders zu unterbinden, diese zeitgesteuerte Abschalteinrichtung durch kurzzeitige Stromunterbrechung zu umgehen, kann im Gehäuse ein Energiespeicher vorgesehen sein, der die Steuereinheit zumindest vorübergehend mit Energie versorgen und die Abschaltung zeitweise aufrechterhalten kann.

Zweckmäßigerweise erfolgt die Energieversorgung des elektrischen Handgeräts über eine externe Energieversorgungseinrichtung. In diesem Fall weist das elektrische Handgerät einen Energieversorgungsanschluss auf, an den insbesondere ein externes Gleichspannungsnetzteil, welches das Handgerät mit einer Gleichspannung versorgen kann, anschließbar ist. Beispielsweise beträgt die Gleichspannung 12V.

Um eine gezielte und effiziente Stromversorgung zu ermöglichen, kann im Gehäuse eine Leiterplatte angeordnet sein, die eine erste Anschlusseinrichtung zum elektrischen Anschließen des elektrisch ansteuerbaren Ventils, eine zweite Anschlusseinrichtung zum elektrischen Anschließen der elektrisch ansteuerbaren Pumpe und eine dritte Anschlusseinrichtung, die elektrisch mit dem Energieversorgungsanschluss und der Steuereinheit verbunden ist, aufweisen, wobei die erste Anschlusseinrichtung, die zweite Anschlusseinrichtung und die dritte Anschlusseinrichtung jeweils über elektrische Leiterbahnen mit der Steuereinheit verbunden sind, und wobei die Steuereinheit dazu ausgebildet ist, das Zuführen einer am Energieversorgungsanschluss anliegenden Gleichspannung an die erste Anschlusseinrichtung zur Energieversorgung des elektrisch ansteuerbaren Ventils und an die zweite Anschlusseinrichtung zur Energieversorgung der elektrisch ansteuerbaren Pumpe zu steuern. Eine solche Leiterplatte kann auch als Steuerplatine bezeichnet werden.

Eine gezielte und effiziente Stromversorgung kann dadurch noch verbessert werden, dass an der Leiterplatte ferner eine vierte Anschlusseinrichtung, die elektrisch mit der Temperaturüberwachungseinrichtung und über Leiterbahnen mit der Steuereinheit verbunden ist, und eine fünfte Anschlusseinrichtung, die elektrisch mit dem Bedienfeld und über Leiterbahnen mit der Steuereinheit verbunden ist, angeordnet sind.

An der Leiterplatte kann eine weitere Anschlusseinrichtung zum elektrischen Anschließen des Vibrationsmotors angeordnet sein, wobei die weitere Anschlusseinrichtung über elektrische Leiterbahnen mit der Steuereinheit verbunden ist, und wobei die Steuereinheit dazu ausgebildet ist, das Zuführen einer am Energieversorgungsanschluss anliegenden Gleichspannung an die Anschlusseinrichtung zur Energieversorgung des Vibrationsmotors zu steuern.

Zudem können der akustische Signalgeber, der Energiespeicher und die lichtemittierenden Bauelemente an der Leiterplatte montiert und über Leiterbahnen mit der Steuereinheit verbunden sein, wobei die Steuereinheit dazu ausgebildet sein kann, die Zuführung einer am Energieversorgungsanschluss anliegenden Gleichspannung auch an die Temperaturüberwachungseinrichtung, das Bedienfeld, den akustischen Signalgeber, die lichtemittierenden Bauelemente und den Energiespeicher zu steuern.

Zweckmäßiger Weise kann die Steuereinheit das elektrisch ansteuerbare Ventil derart ansteuern, dass an der Düse vorzugsweise kontinuierlich erzeugte Druckimpulse zum Beispiel mit definierter Länge und vorzugsweise für eine vorbestimmte Zeit anliegen oder ein im Wesentlichen konstanter Druckstrom beispielsweise für eine vorbestimmte Zeit anliegt. Mittels gepulster Druckinjektion können kosmetische Präparate oder Substanzen ohne Verletzung der Haut in die Hautschichten, insbesondere in die Zellzwischenräume, eingebracht werden. Bei einer Behandlung mit konstantem Druckstrom ist eine sanfte Lymphdrainage insbesondere der Haut der Augenpartie möglich. Auf diese Weise wirkt die konstante Druckstrombehandlung entschwellend.

Angemerkt sei, dass das elektrisch ansteuerbare Ventil vorzugsweise nur in zwei Stellungen, nämlich einer offenen und einer geschlossenen Stellung, betrieben wird.

Bei dem elektrisch ansteuerbaren Ventil handelt es sich vorzugsweise um ein Magnetventil.

Zwar kann die Düse jede beliebige Querschnittsform annehmen, doch läuft die Düse vorzugsweise in Strömungsrichtung trichterförmig auseinander, wobei der Innendurchmesser der Düse zweckmäßigerweise von etwa 0,5 mm bis etwa 25 mm zunehmen kann. Der Öffnungswinkel des Trichters beträgt zweckmäßigerweise weniger als 180°.

Alle zum Betrieb des elektrischen Handgeräts benötigten Parameter, wie zum Beispiel Behandlungszeiten und die zeitliche Dauer eines Druckimpulses, können in einem Speicher abgelegt sein.

Mit dem elektrischen Handgerät soll dem Anwender ein besonders effektives Behandlungsergebnis durch die Kombination aus der Produkteinschleusung mittels mehreren, unmittelbar aufeinanderfolgenden, gepulsten Druckstößen, sowie einer die Hautpartie massierende und entschwellende Behandlung mittels eines konstanten, auf die Hautpartie aufgebrachten Druckstroms, ermöglicht werden. Ergänzend dazu sorgt die während der Behandlung stattfindende Beaufschlagung von Farblicht auf die zu behandelnde Haut und die durch den Vibrationsmotor in Schwingungen versetzte Düse zur Lockerung des Zellverbunds zusätzlich für einen gesteigerten Behandlungseffekt.

Im Gegensatz zur Injektion von Substanzen mittels Nadel oder medizinischem Druckinjektor, mit der zwangsweise eine Verletzung der Haut einhergeht und das Durchdringen der Hautschichten bis in den Blutkreislauf gewünscht ist, erfolgt die Einschleusung von kosmetischen Präparaten in die Haut, insbesondere in die Epidermis und in die darunterliegende Hautschicht, die sogenannte Dermis, mit dem hier vorgeschlagenen elektrischen Handgerät nichtinvasiv und ist dadurch schmerzfrei.

Die Erfindung wird nachstehend anhand eines Ausführungsbeispiels in Verbindung mit den beiliegenden Zeichnungen näher erläutert. Es zeigen:
- Fig. 1: eine Seitenansicht eines beispielhaften elektrischen Handgeräts mit Gehäuse und Düse,
- Fig. 1a: eine perspektivische Ansicht der am Gehäuse des in Fig. 1 gezeigten Handgeräts montierten Düse,
- Fig. 2: als Blockschaltbild des in Fig. 1 gezeigten elektrischen Handgeräts mit geöffnetem Gehäuse,
- Fig. 3: die beispielhaft bestückte Oberseite der in Fig. 2 gezeigten Leiterplatte und
- Fig. 4: die beispielhaft bestückte Unterseite der in Fig. 2 gezeigten Leiterplatte.

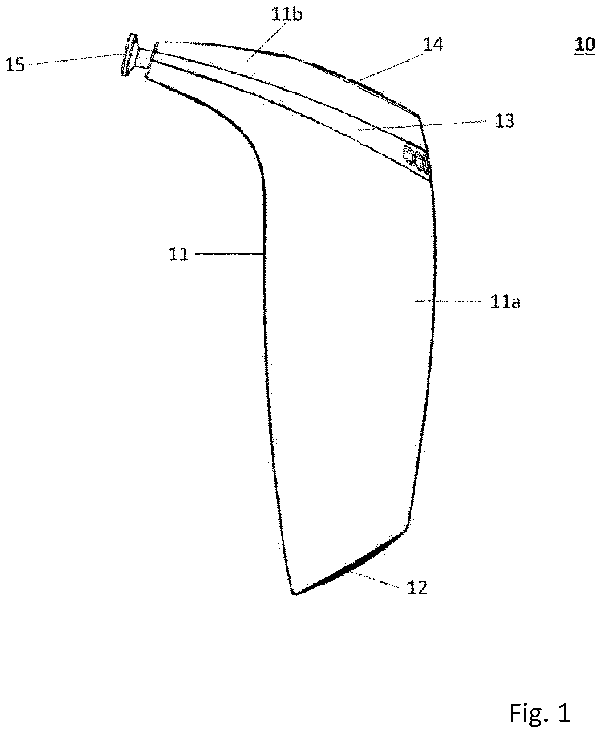

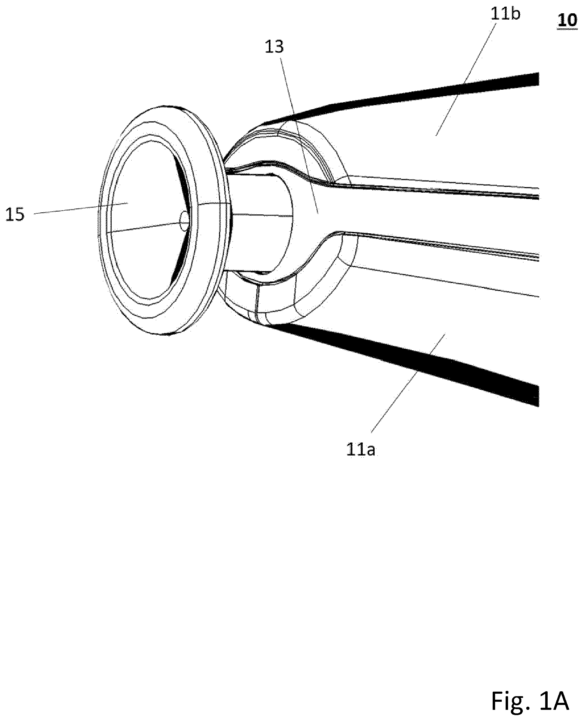

Figur 1 zeigt ein beispielhaftes elektrisches Handgerät 10, welches insbesondere für den Hausgebrauch geeignet ist. Das elektrische Handgerät 10 weist ein Gehäuse 11 auf, an dem eine Düse 15 vorzugsweise lösbar befestigt sein kann. Gehäuse 11 und Düse 15 können aber auch als ein Spritzgussteil hergestellt werden. Das Gehäuse 11 kann, wie gezeigt, im Wesentlichen den Querschnitt eines um 180° gedrehten "L" aufweisen, wobei der längere Schenkel einen Griffteil 11a und der kürzere Schenkel einen Gehäuseteil 11b bildet, an dem die Düse 15 lösbar befestigt sein kann. Das Gehäuseteil 11b kann eine Aussparung aufweisen, in der ein transluzentes Material 13 angeordnet ist. Die Aussparung kann ringförmig verlaufen, so dass das transluzente Material 13 einen Leuchtring bildet. Das transluzente Material 13 ermöglicht u.a. eine Farblichtbehandlung während einer Hautbehandlung, indem beispielsweise blaues Licht, welches von entsprechenden LEDs innerhalb des Gehäuses 11 erzeugt wird, von dem Leuchtring nach vorne, d.h. in Richtung zur Düse 15 auf die zu behandelnde Haut abgestrahlt wird. Wie in Fig. 1a deutlich zu sehen, verläuft das einen Leuchtring bildende transluzente Material 13 auch entlang der Stirnseite des Gehäuseteils 11b, an dem die Düse 15 befestigt ist. Die Düse 15 bzw. deren Schaft erstreckt sich vorzugsweise durch eine Öffnung in dem transluzenten Material 13.

Im Gehäuseteil 11b kann eine weitere Aussparung vorgesehen sein, durch die die Tasten eines Bedienfeldes 14, welches größtenteils im Gehäuse 11 angeordnet ist, betätigt werden können. Das Bedienfeld 14 kann beispielsweise zwei Wahltasten, mit denen zwei Betriebsmodi, zum Beispiel ein "Impulsbetrieb" oder ein "konstanter Druckstrom-Betrieb" eingestellt werden können, und einen Taster zum Ein- und Ausschalten des elektrischen Handgeräts 10 aufweisen. Zusätzlich können die jeweiligen Tasten mit lichtemittierenden Bauteilen zur optischen Unterstützung des Anwenders versehen sein. Im Bodenbereich des Griffteils 11a kann eine Aussparung für einen Energieversorgungsanschluss 12 ausgenommen sein. Der Energieversorgungsanschluss 12 kann als DC-Buchse ausgebildet sein, an die zum Beispiel ein externes 12V DC Steckernetzteil (nicht dargestellt) zur Energieversorgung des elektrischen Handgeräts 10 angeschlossen werden kann.

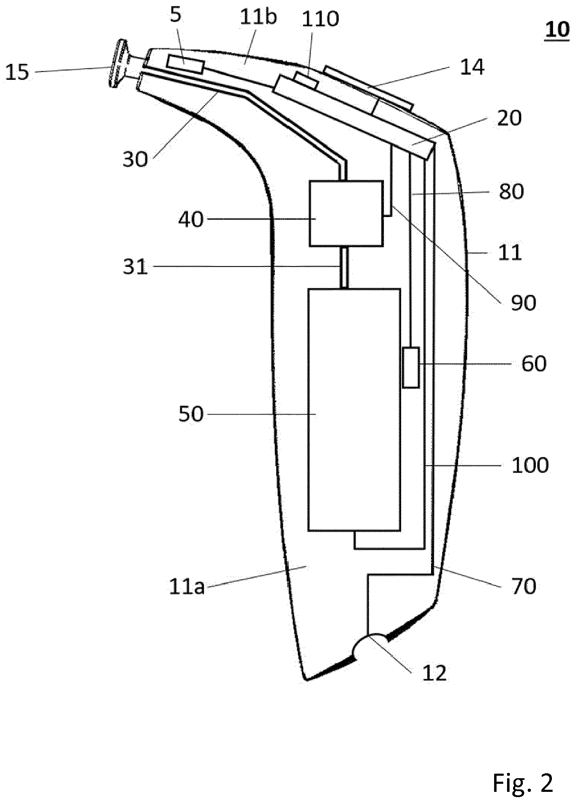

Figur 2 zeigt das in Figur 1 gezeigte beispielhafte elektrische Handgerät 10 mit geöffnetem Gehäuse 11. An der Stirnseite des Gehäuses 11 beziehungsweise des Gehäuseteils 11b ist die Düse 15 vorzugsweise lösbar befestigt. In dem Gehäuse 11 ist eine elektrisch ansteuerbare Pumpe 50 angeordnet, die über einen ersten Druckmittelkanal 31 mit einem elektrisch ansteuerbaren Ventil 40 verbunden ist, welches ebenfalls im Gehäuse 11 angeordnet ist. Es versteht sich, dass auch der erste Druckmittelkanal 31, der beispielsweise als Schlauch ausgebildet sein kann, innerhalb des Gehäuses 11 verläuft. Das elektrisch ansteuerbare Ventil 40 und die elektrisch ansteuerbare Pumpe 50 sind vorzugsweise im Griffteil 11a des Gehäuses 11 untergebracht. In dem Gehäuse 11 ist ein zweiter Druckmittelkanal 30 angeordnet, über den die Düse 15 mit dem elektrisch ansteuerbaren Ventil 40 verbunden ist. Zweckmäßigerweise verläuft der zweite Druckmittelkanal 30, der wiederum ein Schlauch sein kann, im Gehäuseteil 11b.

Eine effiziente Farblichtbehandlung der Haut kann dadurch erzielt werden, dass der zweite Druckmittelkanal 30 aus einem transluzenten Material hergestellt ist, so dass das von den lichtemittierenden Bauelementen ausgesendete blaue Licht durch die Düse 15 direkt auf die zu behandelnde Haut geleitet wird. Beispielsweise ist der zweite Druckmittelkanal 30 als transluzenter Schlauch ausgebildet.

In dem Gehäuseteil 11 kann ein vorzugsweise gleichstrombetriebener Vibrationsmotor 5 angeordnet sein, der dazu ausgebildet ist, die Düse 15 unmittelbar oder mittelbar in Schwingung zu versetzen. Hierzu ist der Vibrationsmotor im Gehäuseteil 11b derart angeordnet, dass er die Düse oder den zweiten Druckmittelkanal 30 oder das den Leuchtring bildende transluzente Material 13 berührt und somit in Schwingung versetzen kann.

Des Weiteren ist in dem Gehäuse 11 eine Steuereinheit 110 angeordnet, die zum Ansteuern des Ventils 40, des Vibrationsmotors 5 und der Pumpe 50 ausgebildet ist. Die Steuereinheit 110 ist insbesondere dazu ausgebildet, das elektrisch ansteuerbare Ventil 40 unter Ansprechen auf einen am Bedienfeld 14 ausgewählten Betriebsmodus derart anzusteuern, dass an der Düse 15 Druckimpulse oder ein im Wesentlichen konstanter Druck anliegen kann. Bei der Steuereinheit 110 kann es sich beispielsweise um einen Mikrokontroller handeln.

Zweckmäßigerweise kann die Steuereinheit 110 die Funktion eines Zeitgebers ausführen. Beispielsweise können in der Steuereinheit 110 oder in einem separaten Speicher (nicht gezeigt) Parameter hinterlegt sein, die zum Beispiel die Zeitdauer eines Druckimpulses und zum Beispiel die Dauer eines Behandlungszyklus, der einige Minuten dauern kann, definieren. In diesem Fall ist die Steuereinheit 110 in der Lage, das elektrisch ansteuerbare Ventil 40 derart anzusteuern, dass an der Düse 15 Druckimpulse mit einer definierten Länge und für eine vorbestimmte Behandlungszeit anliegen oder ein im Wesentlichen konstanter Druck für eine vorbestimmte Behandlungszeit anliegt.

Um das elektrische Handgerät 10 vor Beschädigungen, insbesondere vor einer Überhitzung der elektrisch ansteuerbaren Pumpe 50 zu schützen, kann eine Temperaturüberwachungseinrichtung 60 im Gehäuse 11 angeordnet sein, die elektrisch mit der Steuereinheit 110 verbunden ist. Die Temperaturüberwachungseinrichtung 60 ist vorzugsweise in der Nähe der Pumpe 50 angeordnet, um deren Temperatur zu überwachen. Bei der Temperaturüberwachungseinrichtung 60 kann es sich um einen Temperatursensor handeln, der die gemessene Temperatur zum Beispiel zyklisch der Steuereinheit 110 meldet, die unter Ansprechen auf die Temperatur die elektrisch ansteuerbare Pumpe 50 und/oder das elektrisch ansteuerbare Ventil 40 abschalten kann, sobald ein vorgegebener Temperaturwert überschritten wird. Alternativ kann es sich bei der Temperaturüberwachungseinrichtung 60 auch um einen selbsttätig rückstellenden Temperaturschalter handeln, der bei Überschreiten einer vorgegebenen Temperatur betätigt wird, wobei das Betätigen des Temperaturschalters der Steuereinheit 110 mitgeteilt wird, die daraufhin wiederum die elektrisch ansteuerbare Pumpe 50 und/oder das elektrisch ansteuerbare Ventil 40 abschalten kann. Um ein Überhitzen des elektrisch ansteuerbaren Ventils 40 zu verhindern, kann die Steuereinheit 110 dazu ausgebildet sein, das Ventil 40 mittels einer Pulsweitenmodulation anzusteuern.

Die elektrisch ansteuerbare Pumpe 50, das elektrisch ansteuerbare Ventil 40, der Vibrationsmotor 5, die Temperaturüberwachungseinrichtung 60 und das Bedienfeld 14 sind elektrisch mit der Steuereinheit 110 verbunden.

Um das elektrische Handgerät 10 vorzugsweise mittels einer externen Energieversorgungsquelle mit Energie versorgen zu können, weist das Handgerät 10 den Energieversorgungsanschluss 12 auf. Wie bereits erwähnt, kann an den Energieversorgungsanschluss 12, der vorzugsweise als DC-Buchse ausgebildet ist, zum Beispiel ein externes 12V DC-Steckernetzteil (nicht dargestellt) angeschlossen werden.

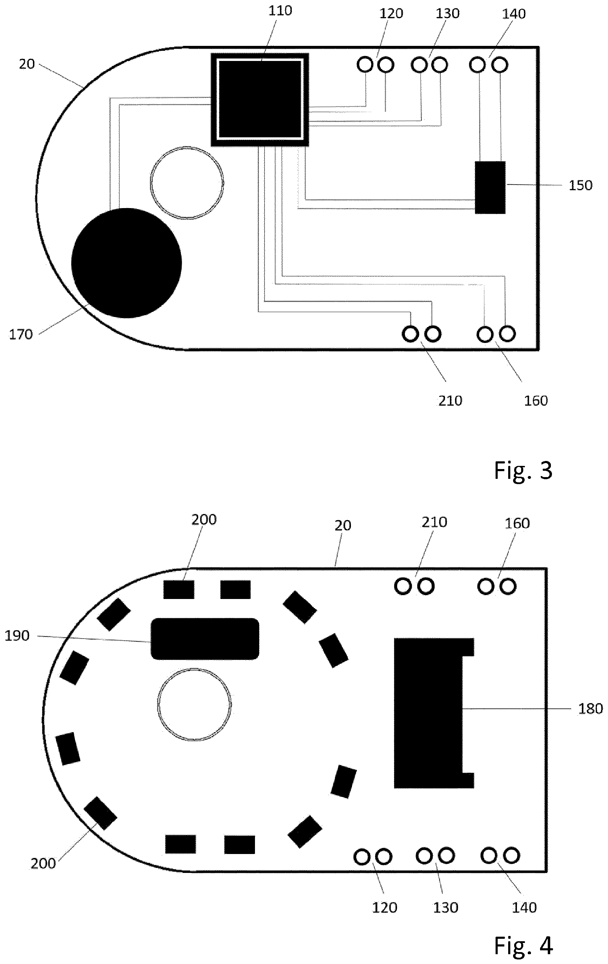

Eine effiziente, kompakte und platzsparende Lösung sieht vor, dass eine Leiterplatte 20 in dem Gehäuse 11, und zwar vorzugsweise im Gehäuseteil 11b angeordnet ist. Die Oberseite der Leiterplatte 20, die vorzugsweise vom Bodenteil des Gehäuses 11 weg weist, ist in Figur 3 dargestellt, während die Unterseite der Leiterplatte 20, die dem Innenraum des Gehäuses 11 zugewandt ist, in Figur 4 dargestellt ist.

Wie in Figur 2 und Figur 3 zu sehen, kann die Steuereinheit 110 auf der Oberseite der Leiterplatte 20 montiert sein.

Weiterhin kann, wie Figur 3 zeigt, die Leiterplatte 20 beispielsweise an der Oberseite eine erste Anschlusseinrichtung 120 zum elektrischen Anschließen des elektrisch ansteuerbaren Ventils 40, eine zweite Anschlusseinrichtung 130 zum elektrischen Anschließen der elektrisch ansteuerbaren Pumpe 50, eine dritte Anschlusseinrichtung 140 zur elektrischen Energieversorgung des Handgerätes 10 und eine weitere Anschlusseinrichtung 210 zum Anschließen des Vibrationsmotors 5 aufweisen.

Gemäß einer beispielhaften Ausgestaltung kann die erste Anschlusseinrichtung 120 zwei Anschlussstellen aufweisen, wobei beide Anschlusspunkte zum Anschließen von zwei Versorgungsleitungen vorgesehen sind, die als elektrische Verbindung 90 zum Ventil 40 führen. Entsprechend sind die beiden Anschlussstellen der Anschlusseinrichtung 120 jeweils über eine Leiterbahn mit der Steuereinheit 110 verbunden, wie dies in Figur 2 und Figur 3 zu sehen ist.

Die Anschlusseinrichtung 210 weist vorzugsweise zwei Anschlussstellen zum Anschließen von zwei Versorgungsleitungen auf, die als elektrische Verbindung zum elektrisch ansteuerbaren Vibrationsmotor 5 führen. Entsprechend sind die beiden Anschlussstellen der Anschlusseinrichtung 210 jeweils über eine Leiterbahn mit der Steuereinheit 110 verbunden, wie dies in Figur 2 und Figur 3 zu sehen ist.

Die zweite Anschlusseinrichtung 130 weist vorzugsweise zwei Anschlussstellen zum Anschließen von zwei Versorgungsleitungen auf, die als elektrische Verbindung 100 zur elektrisch ansteuerbaren Pumpe 50 führen. Entsprechend sind die beiden Anschlussstellen der Anschlusseinrichtung 130 jeweils über eine Leiterbahn mit der Steuereinheit 110 verbunden, wie dies in Figur 2 und Figur 3 zu sehen ist.

Der Energieversorgungsanschluss 12 kann über zwei Anschlussleitungen, die mit Bezugszeichen 70 versehen sind, mit den beiden Anschlussstellen der Anschlusseinrichtung 140 verbunden sein. Die beiden Anschlussstellen der Anschlusseinrichtung 140 sind beispielsweise über eine Sicherung 150 und zwei Leiterbahnen mit der Steuereinheit 110 verbunden.

An der Leiterplatte kann eine vierte Anschlusseinrichtung 160 vorgesehen sein, die beispielsweise wiederum zwei Anschlussstellen aufweist. Die Anschlussstellen der Anschlusseinrichtung 160 können über zwei Anschlussleitungen, die in Figur 2 mit dem Bezugszeichen 80 gekennzeichnet sind, mit der Temperaturüberwachungseinrichtung 60 und über zwei Leiterbahnen mit der Steuereinheit 110 verbunden sein.

Darüber hinaus kann an der Leiterplatte 20 eine fünfte Anschlusseinrichtung 180 vorgesehen sein (siehe Fig. 4), an die das Bedienfeld 14 elektrisch angeschlossen ist und die über Leiterbahnen mit der Steuereinheit 110 verbunden ist. Weiterhin kann ein akustischer Signalgeber 170 im Gehäuse 11 angeordnet sein, der vorzugsweise ebenfalls an der Leiterplatte 20 angeordnet und über entsprechende Leiterbahnen mit der Steuereinheit 110 verbunden ist, wie dies in Figur 3 dargestellt ist.

Die Steuereinheit 110 ist vorzugsweise dazu ausgebildet, gezielt die Zuführung einer am Energieversorgungsanschluss 12 anliegenden Gleichspannung an die Anschlusseinrichtung 120 zur Energieversorgung des elektrisch ansteuerbaren Ventils 40, an die Anschlusseinrichtung 130 zur Energieversorgung der elektrisch ansteuerbaren Pumpe 50, an die Anschlusseinrichtung 160 zur Energieversorgung der Temperaturüberwachungseinrichtung 60, an die Anschlusseinrichtung 180 zur Energieversorgung des Bedienfelds 14, an die Anschlusseinrichtung 210 zur Energieversorgung des Vibrationsmotors 5 und an den akustischen Signalgeber 170 zu steuern. Gemäß dieser beispielhaften Implementierung erfolgt die Energieversorgung der Komponenten des elektrischen Handgeräts 10 folglich nicht unmittelbar über eine am Energieversorgungsanschluss 12 angeschlossenen Energiequelle, sondern mittelbar über die Steuereinheit 110.

Zum Beispiel an der Unterseite der Leiterplatte 20 kann ein Energiespeicher 190, vorzugsweise ein Speicherkondensator, angeordnet sein, der über Leiterbahnen mit der Steuereinheit 110 verbunden ist und über eine am Energieversorgungsanschluss 12 angelegte Gleichspannung aufgeladen werden kann.

Der interne Energiespeicher 190 kann derart dimensioniert sein, dass er nach einer automatischen Abschaltung des Gerätes durch die Steuereinheit 110 bei Erreichen einer vorgegebenen, maximalen Betriebszeit, die sofortige Wiederinbetriebnahme des Gerätes durch den Anwender unterbindet, indem der Energiespeicher 190 die Steuereinheit 110 und damit den Zeitgeber auch im stromlosen Zustand zumindest zeitweise mit Energie versorgen kann.

An der Unterseite der Leiterplatte 20 kann ferner eine Vielzahl von lichtemittierenden Bauelementen 200, die beispielsweise als LEDs ausgebildet sein können, angeordnet sein. Vorzugsweise sind die lichtemittierenden Bauelemente 200 in etwa kreisförmig angeordnet und strahlen vorzugsweise den Hauptanteil des Lichts in Richtung Innenraum des Gehäuses 11, so dass das von den lichtemittierenden Bauelemente 200 ausgesendete Licht über den transluzenten Leuchtring 13 nach außen und vorzugsweise fokussiert in Richtung der Düse 15 ausgestrahlt werden kann. Die lichtemittierenden Bauelemente 200 sind über Leiterbahnen mit der Steuereinheit 110 verbunden. Die Steuereinheit 110 ist dazu ausgebildet, zum Beispiel in Abhängigkeit des Betriebszustands oder des gewählten Betriebsmodus die entsprechenden lichtemittierenden Bauelemente 200 anzusteuern bzw. gezielt mit dem Energieversorgungsanschluss 12 zu verbinden.

Es versteht sich, dass zum Beispiel einige der lichtemittierenden Bauelemente 200 rotes Licht und andere lichtemittierende Bauelemente 200 blaues Licht ausstrahlen können.

Die Steuereinheit 110 kann insbesondere dazu ausgebildet sein, während der Behandlung der Haut die blau leuchtenden, lichtemittierenden Bauelemente 200 anzusteuern, und während eines Fehlerfalls, beispielsweise bei Überhitzung der elektrisch ansteuerbaren Pumpe 50, die rotes Licht ausstrahlenden, lichtemittierenden Bauelemente 200 anzusteuern. Auf diese Weise ist es möglich, dass verschiedene Betriebszustände oder ausgewählte Betriebsmodi des elektrischen Handgeräts 10 unter Steuerung der Steuereinheit 110 optisch dem Anwender signalisiert werden können. In ähnlicher Weise können zum Beispiel verschiedene Betriebszustände dem Anwender über den akustischen Signalgeber 170 und/oder über die in den jeweiligen Tastern des Bedienfeldes 14 integrierten, lichtemittierenden Bauteile, signalisiert werden.

Nachfolgend wird die Funktionsweise des elektrischen Handgeräts 10 erläutert.

Angenommen sei, dass am Energieversorgungsanschluss 12 des Handgeräts 10 eine externe Energieversorgungsquelle angeschlossen und am Bedienfeld 14 das Handgerät 10 eingeschaltet wird. Wird nunmehr der Betriebsmodus "Impulsbetrieb" am Bedienfeld 14 ausgewählt, so sorgt die Steuereinheit 110 beispielsweise dafür, dass die elektrisch ansteuerbare Pumpe 50 und die blau leuchtenden LEDs 200 elektrisch mit dem Energieversorgungsanschluss 12 verbunden und somit mit Strom versorgt werden. Zudem wird das Ventil 40 derart von der Steuereinheit 110 angesteuert, dass es für eine vorbestimmte Behandlungszeit kontinuierlich erzeugte Druckimpulse definierter Länge erzeugt. Hierzu stellt die Steuereinheit 110 über die Anschlussleitung 90 im Rhythmus der gewünschten Druckimpulse die Stromversorgung zum Ventil 40 her oder unterbricht sie und sorgt so dafür, dass das Ventil 40 geöffnet und geschlossen wird.

Zeitgleich kann die Steuereinheit 110 beispielsweise dafür sorgen, dass der Vibrationsmotor 5 elektrisch mit dem Energieversorgungsanschluss 12 verbunden und somit mit Strom versorgt wird. Der Vibrationsmotor 5 versetzt nunmehr die Düse 15 in Schwingung.

Wie in Figur 2 und Figur 3 ersichtlich, ist die Steuereinheit 110 mit dem Energieversorgungsanschluss 12 verbunden. Nunmehr sei angenommen, dass die Temperaturüberwachungseinrichtung 60 betätigt worden ist, da die Temperatur der Pumpe 50 einen kritischen Wert überschritten hat. Unter Ansprechen hierauf trennt die Steuereinheit 110 die elektrisch ansteuerbare Pumpe 50 und/oder das elektrisch ansteuerbare Ventil 40 vom Energieversorgungsanschluss 12 und somit von der externen Energieversorgungsquelle. Je nach Implementierung kann die Steuereinheit 110 derart programmiert sein, dass sie verhindern kann, dass die Pumpe 50 und/oder das Ventil 40 nach Abschaltung infolge einer Überhitzung vor Ablauf einer ausreichenden Abkühlphase, nämlich beispielsweise bis zum Rückstellen des Temperaturschalters, wieder eingeschaltet werden.

Damit die Steuereinheit 110 außerdem die maximal zulässige, bestimmungsgemäße Betriebsdauer des Gerätes auch dann überwachen kann, wenn das elektrische Handgerät 10, wenn auch zum Beispiel nur kurzzeitig, von der externen Energieversorgungsquelle getrennt wird, kann der interne Energiespeicher 190 die Steuereinheit 110 zumindest zweitweise mit ausreichender Energie versorgen, um eine sofortige Wiederinbetriebnahme des Gerätes durch den Anwender zu blockieren.

## Patentansprüche

1. Elektrisches Handgerät (10) zum Einbringen von kosmetischen Präparaten unter Druck in die Haut, aufweisend:
ein Gehäuse (11) und eine auf eine zu behandelnde Hautstelle aufsetzbare Düse (15), wobei in dem Gehäuse (11)
mehrere im Gehäuse (11) angeordnete lichtemittierende Bauelemente (200), die zum Erzeugen von blauem Licht ausgebildet sind,
eine elektrisch ansteuerbare Pumpe (50),
ein elektrisch ansteuerbares Ventil (40), welches über einen ersten Druckmittelkanal (31) mit der elektrischen Pumpe (50) verbunden ist,
ein zweiter Druckmittelkanal (30), und
eine Steuereinheit (110) zum Ansteuern des elektrisch ansteuerbaren Ventils (40) und der elektrisch ansteuerbaren Pumpe (50) angeordnet sind, und wobei die Düse (15) über den zweiten Druckmittelkanal (30) mit dem elektrisch ansteuerbaren Ventil (40) verbunden ist,
ein transluzentes Material (13) zur Farblichtbehandlung der zu behandelnden Haut, wobei das transluzente Material (13) in einer ringförmigen Aussparung des Gehäuses (11) angeordnet ist, und
wobei die Steuereinheit (110) zum Ansteuern der lichtemittierenden Bauelemente (200) ausgebildet ist.

2. Elektrisches Handgerät nach Anspruch 1,
**gekennzeichnet durch**
eine im Gehäuse (11) angeordnete Temperaturüberwachungseinrichtung (60), die elektrisch mit der Steuereinheit (110) verbunden ist.

3. Elektrisches Handgerät nach Anspruch 1 oder 2,
**gekennzeichnet durch**
ein elektrisch mit der Steuereinheit (110) verbundenes Bedienfeld (14) zur Eingabe von Steuerbefehlen.

4. Elektrisches Handgerät nach einem der vorstehenden Ansprüche,
**gekennzeichnet durch**
einen im Gehäuse (11) angeordneten Vibrationsmotor (5), der dazu ausgebildet ist, die Düse (15) in Schwingung zu versetzen.

5. Elektrisches Handgerät nach einem der vorstehenden Ansprüche,
**gekennzeichnet durch**
einen akustischen Signalgeber (170), der elektrisch mit der Steuereinheit (110) verbunden ist.

6. Elektrisches Handgerät nach einem der vorstehenden Ansprüche,
**gekennzeichnet durch**
einen im Gehäuse angeordneten Energiespeicher (190), der die Steuereinheit (110) vorübergehend mit Energie versorgen kann.

7. Elektrisches Handgerät nach einem der vorstehenden Ansprüche,
**gekennzeichnet durch**
einen Energieversorgungsanschluss (12), an den ein externes Gleichspannungsnetzteil, welches das elektrische Handgerät (10) mit einer Gleichspannung versorgen kann, anschließbar ist.

8. Elektrisches Handgerät nach Anspruch 7,
**gekennzeichnet durch**
eine im Gehäuse (11) angeordnete Leiterplatte (20) mit einer ersten Anschlusseinrichtung (120) zum elektrischen Anschließen des elektrisch ansteuerbaren Ventils (40),
einer zweiten Anschlusseinrichtung (130) zum elektrischen Anschließen der elektrisch ansteuerbaren Pumpe (50),
einer dritten Anschlusseinrichtung (140), die elektrisch mit dem Energieversorgungsanschluss (12) und der Steuereinheit (110) verbunden ist, wobei die erste Anschlusseinrichtung (120), die zweite Anschlusseinrichtung (130) und die dritte Anschlusseinrichtung (140) jeweils über elektrische Leiterbahnen mit der Steuereinheit (110) verbunden sind, und wobei die Steuereinheit (110) dazu ausgebildet ist, das Zuführen einer am Energieversorgungsanschluss (12) anliegenden Gleichspannung an die erste Anschlusseinrichtung (120) zur Energieversorgung des elektrisch ansteuerbaren Ventils (40) und an die zweite Anschlusseinrichtung (130) zur Energieversorgung der elektrisch ansteuerbaren Pumpe (50) zu steuern.

9. Elektrisches Handgerät nach den Ansprüchen 2 bis 6 und 8,
**dadurch gekennzeichnet, dass**
an der Leiterplatte (20) eine vierte Anschlusseinrichtung (160) vorgesehen ist, die elektrisch mit der Temperaturüberwachungseinrichtung (60) und über Leiterbahnen mit der Steuereinheit (110) verbunden ist, dass
an der Leiterplatte (20) eine fünfte Anschlusseinrichtung (180) vorgesehen ist, die elektrisch mit dem Bedienfeld (14) und über Leiterbahnen mit der Steuereinheit (110) verbunden ist, dass
der akustische Signalgeber (170), der Energiespeicher (190) und die lichtemittierenden Bauelemente (200) an der Leiterplatte (20) montiert und über Leiterbahnen mit der Steuereinheit (110) verbunden sind, und dass die Steuereinheit (110) dazu ausgebildet ist, das Zuführen einer am Energieversorgungsanschluss (12) anliegenden Gleichspannung an die Temperaturüberwachungseinrichtung (60), das Bedienfeld (14), den akustischen Signalgeber (170), die lichtemittierenden Bauelemente (200) und den Energiespeicher (190) zu steuern.

10. Elektrisches Handgerät nach einem der vorstehenden Ansprüche,
**dadurch gekennzeichnet, dass**
die Steuereinheit (110) die Funktion eines Zeitgebers ausführen kann.

11. Elektrisches Handgerät nach einem der vorstehenden Ansprüche,
**dadurch gekennzeichnet, dass**
die Steuereinheit (110) das elektrisch ansteuerbare Ventil (40) derart ansteuern kann, dass an der Düse (15) Druckimpulse definierter Länge für eine vorbestimmte Zeit anliegen oder ein im Wesentlichen konstanter Druckstrom für eine vorbestimmbare Zeit anliegt.

12. Elektrisches Handgerät nach Anspruch 4,
**dadurch gekennzeichnet, dass**
die Leiterplatte (20) eine Anschlusseinrichtung (210) zum elektrischen Anschließen des Vibrationsmotors (5) aufweist, wobei die Anschlusseinrichtung (210) über elektrische Leiterbahnen mit der Steuereinheit (110) verbunden ist, und wobei die Steuereinheit (110) dazu ausgebildet ist, das Zuführen einer am Energieversorgungsanschluss (12) anliegenden Gleichspannung an die Anschlusseinrichtung (210) zur Energieversorgung des Vibrationsmotors (5) zu steuern.

13. Elektrisches Handgerät nach einem der vorstehenden Ansprüche,
**dadurch gekennzeichnet, dass**
der zweite Druckmittelkanal (30) aus einem transluzenten Material hergestellt ist, so dass das von den lichtemittierenden Bauelementen ausgesendete blaue Licht durch die Düse (15) auf die zu behandelnde Haut geleitet wird.
